# EUROPEAN PATENT APPLICATION

(11) **EP 3 407 164 A1**
(43) Date of publication of application: **28.11.2018**
(21) Application number: 17172408.1
(22) Date of filing: 23.05.2017
(51) Int. Cl.: G06F 3/01, A61B 5/0496, G06F 3/038, G02B 27/01

(54) **A METHOD AND A SYSTEM FOR MONITORING AN EYE POSITION**

(71) Applicant: Stichting IMEC Nederland, 5656 AE Eindhoven (NL)
(72) Inventor: AGELL, Carlos, 3001 Leuven (BE); CASALE, Pierluigi, 3001 Leuven (BE); SQUILLACE, Gabriel, 3001 Leuven (BE)
(74) Representative: AWA Sweden AB

(57) **Abstract**

A method for monitoring an eye position, comprises: capturing (202) a sequence of digital images of an eye; acquiring (204) a sequence of biosignal data representing eye movements; determining (206) a set of reference eye positions based on the sequence of digital images; and determining (208) a set of intermediate eye positions based on said set of reference eye positions and said sequence of biosignal data, said set of intermediate eye positions representing eye positions relative to said set of reference eye positions, wherein the set of intermediate eye positions represents eye positions between consecutive pairs of images of said sequence of digital images.

## Description

### Technical field

The present inventive concept relates to a method and a system for monitoring an eye position.

### Background

Eye tracking may be based on using pairs of electrodes to detect potential differences around an eye, which may be converted to an eye movement. The electrodes in a pair may be placed on opposite sides of an eye for measuring corneo-retinal standing potential that exists between front and back of a human eye, often referred to as electrooculography (EOG), and the detected potential difference may be related to an eye position. The electrodes may also or alternatively be arranged to acquire muscle-generated biopotential signals so as to detect eye movements.

Such biosignal-based eye tracking may be advantageously used in detecting eye movements and is often used in eye tracking applications, such as read speed analysis and sleep analysis. The detection of a potential difference may be very fast, which allows the biosignal-based eye tracking to detect fast eye movements. However, the detection of a potential difference may produce drift errors so that it is not possible to accurately determine the eye position over a period of time, but merely differential information such as eye movements may be detected.

### Summary

An objective of the present inventive concept is to enable improved eye tracking. Additional objectives include enabling a more reliable and accurate eye tracking, while still able to detect fast eye movements. Further and alternative objectives may be understood from the following.

According to a first aspect, there is provided a method for monitoring an eye position, comprising: capturing a sequence of digital images of an eye; acquiring a sequence of biosignal data representing eye movements; determining a set of reference eye positions based on the sequence of digital images; and determining a set of intermediate eye positions based on said set of reference eye positions and said sequence of biosignal data, said set of intermediate eye positions representing eye positions relative to said set of reference eye positions, wherein the set of intermediate eye positions represents eye positions between consecutive pairs of images of said sequence of digital images.

Thanks to capturing a sequence of digital images of an eye, accurate eye positions may be determined based on the digital images by using image processing techniques. Hence, reference eye positions may be determined and acquired biosignal data may be related to the reference eye positions for determining eye positions intermediate to the reference eye positions. Thus, accuracy of eye positions determined on biosignal data may be improved such that the biosignal data may be reliably used as indication of eye position and not only for detection of eye movements.

A speed of capturing and processing of digital images of an eye may be limited by a frame rate of the image capturing device, such that accurate reference positions may not be provided at very short intervals. However, the use of acquired biosignal data acquired at much higher rate than the frame rate of the image capturing device enables determining of eye positions also between two time instances corresponding to the capturing time of two consecutive digital images. The biosignal data may thus provide intermediate eye positions between the accurate eye positions which may be determined based on the digital images.

An eye position, as used herein, refers to a position, or equivalently the orientation or the rotation, of the eye in relation to a frame of reference, e.g. in relation to the head of the user (i.e. a frame of reference having a fixed relationship with respect to the head). The position of the eye may determine the gaze direction in relation to the frame of reference. The eye position may thus define a position of the pupil in the eye in relation to a frame of reference based on the head of the user. However, the eye position may alternatively define a position of the eye corresponding to a gaze direction, such that the eye position may be represented as an angle corresponding to the gaze direction. As a further alternative, the eye position may be defined in a frame of reference being viewed by the user, such that a gaze direction may be represented in form of a position in a frame of reference, such as a screen, which the user looks at.

The method for monitoring an eye position may be performed on both eyes of a user. Thus, each eye may be separately monitored in order to determine the eye position of the respective eye. However, it should also be realized that, when monitoring the eye positions of both eyes of a user, the information from the respective monitoring of eye positions may be combined and further conclusions of a gaze direction of the user may be drawn. For instance, by determining gaze directions of each eye, the gaze directions may be compared in order to determine a distance from a user to an object at which the user is looking. If the user is looking at an object that is close to the user, angles of the gaze directions of the respective eyes may form large converging opposite angles, whereas if the user is looking at an object far away, the gaze directions may form close to parallel angles.

A reference eye position may set a reference or "ground truth" indicating an eye position at a specific instant in time. Biosignal data following in time may then be used for determining changes of the eye position in relation to the reference, such that eye positions may be determined based on biosignal data in time instances following the specific instant in which the reference eye position was determined.

The method may be used for monitoring eye positions in real time or near real time and the eye positions may thus be used e.g. as real time input, which could for example be used for controlling a computer. The steps of determining reference eye positions and intermediate eye positions could also or alternatively be used in a separate processing operation, which need not be performed in real time. Thus, the capturing of digital images and acquiring of biosignal data may collect sample information, which may be used in later processing, e.g. for performing advanced analysis of eye movements which may not be suitable to be performed in real time.

When performing a separate processing operation on a sequence of digital images and a sequence of biosignal data, the information in the sequences may be considered as a whole and need not necessarily be processed in the time order of capturing and acquiring the information. For instance, when determining intermediate eye positions, both reference eye positions of a consecutive pair of images may be used as a basis for determining intermediate eye positions such that the biosignal data representing intermediate eye positions corresponds to a movement of the eye position from a first reference position represented by a first image in the consecutive pair to a second reference position represented by a second image in the consecutive pair.

According to an embodiment, the method further comprises determining a set of relative eye positions based on said sequence of biosignal data, wherein said determining of a set of intermediate eye positions comprises combining said set of reference eye positions and said set of relative eye positions.

Hence, the biosignal data may be processed to determine relative eye positions forming a sequence of relative positions. This implies that the biosignal data may be separately processed, e.g. in a separate processor or processing thread, in order to determine the set of relative eye positions, such that the biosignal data can be processed while a reference eye position has not yet been determined based on a previous digital image. Once a reference eye position has been determined, the set of relative eye positions may then be easily combined with the reference eye position in order to enable absolute positions to be formed based on each of the relative eye positions. Thus, the determining of the set of relative eye positions based on the sequence of biosignal data facilitates separation of processing of the digital images and the biosignal data, which may be highly advantageous in real time monitoring of an eye position.

According to an embodiment, the method further comprises, in a combining unit, receiving a first stream comprising the determined set of reference eye positions, receiving a second stream comprising the determined relative eye positions, and combining the first and second streams into a single stream of eye positions. Thus, a combining unit may form a single stream of eye positions, which are reliable and may be provided at a high rate facilitating use of the monitoring of eye positions in real time applications. The first and second streams may share a time reference or the combining unit may know how to relate relative eye positions in time to the reference eye positions in order to properly combine the first and second streams into a single stream of consecutive eye positions.

According to an embodiment, a sample rate of the second stream is higher than a sample rate of the first stream, and wherein the combined single stream has a sample rate higher than the sample rate of the first stream. Thus, the sample rate of eye positions output from the combining unit may be higher than a rate of capturing digital images. The sample rate of the single stream may correspond to the sample rate of the second stream, which may be substantially higher than the sample rate of the first stream. However, the sample rate of the single stream may alternatively be between the sample rate of the first and second streams, such as half the sample rate of the second stream. Although a single stream may have a lower rate than the second stream, all viable samples of the second stream may still be used in determining the intermediate positions included in the single stream. It should be realized that some samples may not contribute to providing a correct eye position, e.g. digital images of an eye when the eyelid is closed or biosignal data in relation to a blink or artifact. Such samples could be excluded or disregarded from a stream when combining the first and second streams to a single stream.

According to an embodiment, the set of reference eye positions and the set of relative eye positions are determined in relation to a common coordinate system. Thus, the reference eye positions and the relative eye positions may be easily combined as they may be represented in a common coordinate system.

According to an embodiment, the common coordinate system is based on an external reference. This implies that the eye positions may be related to an external reference. In one embodiment, the external reference may correspond to a screen at a controlled distance from a user's head, and the eye positions may be represented as positions on the screen at which the eye is directed. A supporting or guiding structure may be used in order to maintain a controlled, constant distance between the screen and the user's head. The screen may also be mounted in relation to the user's head, such as being arranged in goggles worn by the user, whereby a controlled distance between the screen and user's head is ensured.

According to an embodiment, the method further comprises calibrating eye positions based on digital images of an eye to eye positions based on biosignal data, said calibrating comprising providing a sequence of stimuli trigging a sequence of predictable eye positions, capturing a calibration sequence of digital images of an eye in relation to the sequence of predictable eye positions; acquiring a calibration sequence of biosignal data representing eye movements in relation to the sequence of predictable eye positions, and determining calibration data for the reference eye positions and calibration data for the intermediate eye positions in a common coordinate system. The calibration may thus relate image-based eye positions to biosignal-based eye positions, such that reference eye positions and intermediate eye positions are calibrated based on common predictable eye positions, which enables calibration data to be determined in relation to a common coordinate system. This facilitates combining of image-based eye positions with biosignal-based eye positions.

According to an embodiment, said sequence of stimuli comprises indications of screen positions and wherein said calibration data correlates reference eye positions and intermediate eye positions to screen positions. Hence, the eye positions may be related to the screen, which may facilitate controlling a processing unit by interaction with information presented on the screen.

The calibration may be performed with a controlled distance between the user's head and the screen. The calibration may thus ensure that eye positions may be represented in the form of positions on the screen for a user's head being at a controlled distance to the screen. However, it should also be realized that multiple calibrations may be performed in order to provide calibration data in relation to different distances between the user's head and the screen.

According to a second aspect, there is provided a system for monitoring an eye position, comprising: an image capturing device configured to capture a sequence of digital images of an eye; a biosignal acquisition unit configured to acquire a sequence of biosignal data representing eye movements; a processing unit configured to determine a set of reference eye positions based on the sequence of digital images; and determine a set of intermediate eye positions based on said set of reference eye positions and said sequence of biosignal data, said set of intermediate eye positions representing eye positions relative to said set of reference eye positions, wherein the set of intermediate eye positions represents eye positions between consecutive pairs of images of said sequence of digital images.

Effects and features of this second aspect are largely analogous to those described above in connection with the first aspect. Embodiments mentioned in relation to the first aspect are largely compatible with the second aspect.

The system may thus include an image capturing device, a biosignal acquisition unit and a processing unit, which may be operated to generate reference eye positions and intermediate eye position. Hence, the system may generate eye positions with a high sample rate, while still providing reliable and accurate eye positions.

The image capturing device, the biosignal acquisition unit and the processing unit may each be a separate device and may be arranged to communicate with each other using wired or wireless communication. However, one or more of the components may be integrated in a common housing, which may facilitate handling of the system. For instance, relations between the components may be pre-defined in the housing, such that set-up of the system before first use may be simplified.

By image capturing device is hereby meant any device having the function of imaging, in the form of digital image data. The image capturing device may be a digital camera or any imaging sensor (complementary metal-oxide-semiconductor (CMOS) or a charge-coupled device (CCD)) with digital readout.

By biosignal acquisition unit is here meant any unit being capable of acquiring analog biosignals by electrical measurements on the user, preferably via a set of skin electrodes. The biosignal acquisition unit may further convert the analog biosignals to digital samples. The unit may be a dedicated sensor circuit, an application specific integrated circuit (ASIC) or a block of a higher functionality system, such as a system on chip (SoC) or system in package (SiP).

According to an embodiment, the processing unit comprises an image processor configured to determine a set of reference eye positions based on the sequence of digital images and a biosignal processor configured to determine a set of relative eye positions based on said sequence of biosignal data. Hence, the sequence of digital images and the sequence of biosignal data may be separately processed. Thus, for instance, the biosignal data can be processed while a reference eye position has not yet been determined based on a previous digital image.

The processing unit may comprise dedicated processor blocks, such that separate image processor and biosignal processor may be provided. However, the processing unit could alternatively comprise a single processing unit, such as a central processing unit (CPU), which may execute separate processing threads for the image processor and the biosignal processor. Also, processor blocks for implementing the image processor and the biosignal processor may be arranged anywhere in the system, and even embedded in the image capturing device and the biosignal acquisition unit, respectively.

According to an embodiment, the processing unit further comprises a combiner configured to determine intermediate eye positions based on said set of reference eye positions and said set of relative eye positions. Hence, the relative eye positions may be converted to absolute positions based on the reference eye positions.

According to an embodiment, the combiner is configured to output a single stream of eye positions based on said set of reference eye positions and said set of relative eye positions. Thus, a single stream of eye positions may be provided and the single stream may be easily used by e.g. an external unit, which may react to input on eye positions.

According to a third aspect, there is provided a system for controlling presentation on a screen, comprising: a screen configured to present information to a user; a system for monitoring an eye position according to the second aspect, wherein the eye positions are determined in relation to positions on the screen; and a controller configured to receive the eye positions as indications of gaze directions of a user and further configured to control the screen in relation to the received eye positions.

Effects and features of this third aspect are largely analogous to those described above in connection with the first and second aspects. Embodiments mentioned in relation to the first and second aspects are largely compatible with the third aspect.

The system for monitoring an eye position may thus be integrated with a system for controlling presentation on a screen, such that information presented on the screen may be at least partly controlled by eye movements. This may be very useful e.g. for allowing disabled persons to control a computer or for allowing eye-control of any processing device in a virtual or augmented reality system.

According to a fourth aspect, there is provided a device for monitoring an eye position, comprising: a carrier configured to be head-mounted on a user; and the system according to the second aspect, wherein the system is mounted on the carrier.

Effects and features of this fourth aspect are largely analogous to those described above in connection with the first, second, and third aspects. Embodiments mentioned in relation to the first, second, and third aspects are largely compatible with the fourth aspect.

Thanks to mounting of the system on a carrier, which is configured to be head-mounted, the device for monitoring an eye position may be very easy to use. The image capturing device and the biosignal acquisition unit may be arranged on the carrier in such a way that the image capturing device and the biosignal acquisition unit will be arranged in a proper relationship to an eye of the person, when the head-mounted carrier is worn. Hence, the system may be ready to use as soon as the head-mounted carrier is arranged on a user's head.

According to an embodiment, the carrier is a pair of glasses or a headset for virtual or augmented reality. Hence, the device for monitoring an eye position may be integrated into a carrier, which may be anyway be worn by a user for providing virtual or augmented reality. The eye positions determined by the system may then be used as input for control of the virtual or augmented reality.

It should be realized that the carrier, e.g. when implementing a virtual or augmented reality, may also provide a built-in screen which is arranged at a well-controlled distance to the user. Stimuli for calibration may thus be projected on the built-in screen and corresponding calibration sequences of digital images and biosignal data may be acquired for calibrating eye positions to a coordinate system of the built-in screen.

### Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1 is a block diagram of a system for monitoring an eye position according to an embodiment.
Fig. 2 is a view of a user illustrating placement of electrodes of for acquisition of biosignal data.
Fig. 3 is a flowchart of a method according to an embodiment.
Fig. 4 is a schematic view of combining image-based data and biosignal data to a stream of eye positions.
Fig. 5 is a chart illustrating combination of data.
Fig. 6 is a schematic view illustrating calibration of the system of Fig. 1.
Fig. 7 is a chart illustrating biosignal data and image-based data and forming a single stream of eye positions.
Fig. 8 is a schematic view of a system for controlling presentation on a screen.
Fig. 9 is a schematic view of a device having a head-mounted carrier on which the system for monitoring an eye position is mounted.

### Detailed description

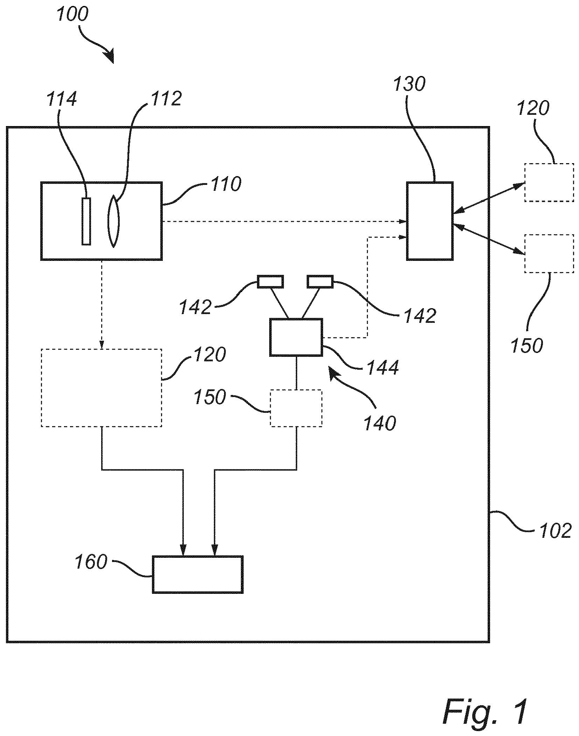

Referring now to Fig. 1, a system 100 for eye tracking will be discussed. Eye tracking may be used for multiple applications, such as in research of the human visual system or as input for controlling interaction between a human and a computing device. For instance, the system 100 may at least partly be implemented in a wearable device, e.g. in a head-mounted structure such as goggles, or other eyewear that are worn by a user in applications ranging from augmented reality, virtual reality or biosignal acquisition and processing. A pair of goggles may be provided with two systems 100 for eye tracking, one system 100 for each eye. However, two such systems 100 may at least partly share components, for instance, for processing acquired data. Below, only a single system 100 will be described.

The system 100 may comprise an image capturing device 110. The image capturing device 110 may be implemented as a digital camera, which may be integrated in a wearable device. For instance, the camera may be arranged in the head-mounted structure worn by the user, set up to acquire images from the user's eyes in a close range. However, the image capturing device 110 may also be arranged at a distance from the user. For instance, the image capturing device 110 may be formed by a digital camera integrated in or connectable to a desktop computer monitor, a laptop, a mobile phone, a tablet computer or some other portable computing device. Other examples include a TV or a video game console.

The image capturing device 110 may comprise an optical system 112 and an image sensor 114. The optical system 112 may be arranged to image an object onto the image sensor 114. The optical system 112 may be preconfigured to be adapted for imaging an eye in close range. For instance, a distance between the optical system 112 and an eye may be well-known in advance, if the image capturing device 110 is integrated in the head-mounted structure, such as goggles.

The image sensor 114 may comprise an array of photo-sensitive areas and may be arranged to record an image by means of the photo-sensitive areas being controlled to output signals representative of accumulated incoming light.

The image sensor 114 may be a complementary metal-oxide-semiconductor (CMOS) image sensor or a charge-coupled device (CCD) image sensor.

The image capturing device 110 may be configured to capture a sequence of digital images of an eye. The images may be arranged to image the eye, and possibly a small area around the eye in order to allow determining an eye position of a user which may be indicative of a gaze direction and possibly other eye features providing useful information, such as pupil location, pupil area, pupil speed, unique iris identification information, and reaction time to optical stimuli.

The system 100 may further comprise an image processing unit 120. The image processing unit 120 may be configured to receive data including the sequence of digital images from the image capturing device 110.

The image processing unit 120 may be a logic digital block of a higher level entity such as an ASIC, SiP, SoC, intrinsically connected to the image sensor 114, e.g. by sharing a data bus.

The image processing unit 120 may be directly connected to the image sensor 114, e.g. by being mounted on a common printed circuit board or connected through a wired connection to the image sensor 114.

Alternatively, the image processing unit 120 may be arranged remotely to the image capturing device 110. For instance, the image processing unit 120 may be arranged in a desktop computer, a laptop, a TV, a video game console or in a portable computing device, which may also be carried or worn by the user, such as in a mobile phone or a tablet computer. In such case, the system 100 may further comprise a transmitter 130 for communicating between the image capturing device 110 and the image processing unit 120. For instance, the transmitter 130 may be arranged for wireless communication, e.g. using Bluetooth®/WiFi® or another wireless protocol, with an external unit in which the image processing unit 120 may be arranged.

The image processing unit 120 may be configured to process the sequence of digital images in order to determine a sequence of positions, orientations, rotations and other features of the eye. The image processing unit 120 may, for instance, determine a position of the pupil and/or a position of the iris, the area of the pupil, its perimeter, or the ratio between areas of iris and pupil which may in turn be used to determine a gaze direction of the user, a reaction of the user to external stimuli or the eye speed, among other eye-related features.

The processing by the image processing unit 120 may include further eye feature extraction. For instance, pupil size and iris measurements may be performed for each digital image. Also, based on the sequence of digital images, eye feature extraction may include eye movement, pupil variation, pupil velocity, etc.

The image processing unit 120 may be any unit being capable of performing digital image processing. The image processing unit 120 may be implemented as a dedicated image processing unit 120 including circuitry dedicated to performing the functions of the image processing unit 120. The circuit may be a digital logic circuit. The circuit may be implemented in an integrated circuit such as a chipset. The circuit may also be implemented in a field programmable gate array (FPGA) or an application specific integrated circuit (ASIC). The image processing unit 120 may also be implemented in a general-purpose processing unit, such as a microprocessor, e.g. a central processing unit (CPU), which may be provided with a set of software instructions for performing the processing operations.

The features extracted by the image processing unit 120 may be stored in a memory for future analysis and/or may be reported, e.g. to a controller for interpreting the extracted features in providing a human-computer interface.

The image processing unit 120 may need to perform relatively extensive image processing of each image in order to extract the desired features. The image processing performed by the processing unit 120 may thus be based on an assumption that an eye is imaged in each digital image in order to speed up processing.

The system 100 may further comprise a biosignal acquisition unit 140. The biosignal acquisition unit 140 may be configured to acquire biosignal data, which may represent an eye activity. In this respect, the biosignal acquisition unit 140 may be arranged to register biopotentials based on muscle, skin or nerve activity in relation to eye activity.

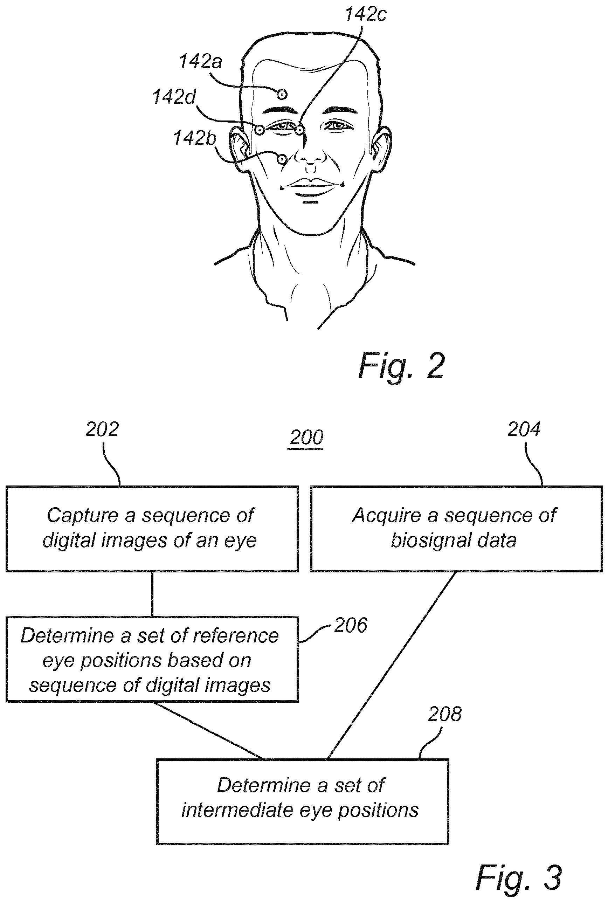

The biosignal acquisition unit 140 may comprise a set of skin electrodes 142 adapted to be arranged in an eye region of the user. The set of skin electrodes 142 may comprise a pair of skin electrodes 142a-b, which are arranged above and below an eye, respectively, as illustrated in Fig. 2. Additionally, the biosignal acquisition unit 140 may comprise a pair of skin electrodes 142c-d, which are arranged to the left and right of an eye, respectively.

The eye acts as a dipole in which the positive pole is at the cornea and the negative pole is at the retina. A potential difference between the electrodes 142 may be representative of an arrangement of the eye dipole in relation to the skin electrodes 142. Biosignal data acquired based on detecting an arrangement of the eye dipole in relation to the skin electrodes 142 may be called electrooculography (EOG) data. The biosignal data may be indicative of eye movements and detection of EOG data may thus be used for determining eye movements, e.g. as a sequence of relative eye positions.

A first pair of electrodes 142a-b arranged above and below the eye may thus be arranged to determine eye movement in relation to a vertical direction, whereas a second pair of electrodes 142c-d arranged to the left and right of the eye may be arranged to determine eye movement in relation to a horizontal direction. Using both pairs of electrodes, horizontal and vertical direction movements may be separately detected and together represent a movement of the eye in two dimensions.

As mentioned above, two parallel systems 100 may also be set up to monitor the position of both eyes of a user. In such case, the biosignal acquisition units 140 of the parallel systems 100 may comprise separate sets of electrodes, each set being intended for monitoring one eye. However, it should also be realized that at least one of the electrodes may be used for monitoring a position of both eyes. For instance, a single electrode 142a may be arranged above both eyes (extending over a larger area than indicated in Fig. 2) and then a first electrode 142b below the right eye of the user may be configured to detect a biopotential in relation to the single electrode 142a as a measure of eye movements of the right eye, whereas a second electrode (not shown) below the left eye of the user may be configured to detect a biopotential in relation to the single electrode 142a as a measure of eye movement of the left eye. Similarly, an electrode arranged between the eyes may be shared and used for acquiring a measure of horizontal eye movements in relation to electrodes arranged to the right of the right eye of the user and to the left of the left eye of the user (as seen from the user's perspective).

The pairs of electrodes 142a-b, 142c-d need not be perfectly aligned on a vertical and horizontal axis, respectively, in relation to the eye. Rather, the measure acquired based on the respective pairs of electrodes 142a-b, 142c-d may then have a component in the vertical and horizontal direction, respectively and a geometrical correction may be performed on the acquired signals in order to obtain orthogonal projections belonging to the horizontal and vertical axis. This may be especially useful when having two parallel systems 100 monitoring the positions of both eyes of the user.

The biosignal acquisition unit 140 may further comprise circuitry 144 connected to the set of skin electrodes 142 and arranged to measure the potential difference between the skin electrodes 142 in each pair of skin electrodes 142a-b, 142c-d and acquire the measured potential difference as biosignal data by sampling and digitizing the measured potential difference. Circuitry for measuring and acquiring of data of a potential difference is per se known in the art and will therefore not be further described herein.

The system 100 may further comprise a biosignal processing unit 150. The biosignal processing unit 150 may be configured to receive the biosignal data from the biosignal acquisition unit 140.

The biosignal processing unit 150 may be configured to process the received biosignal data in order to determine eye positions based on an arrangement of the eye dipole and/or based on detected muscle activity which may be correlated to movements of the eye. The biosignal processing unit 150 may thus comprise an algorithm for analyzing the biosignal data so as to determine positions of the eye, at least relative positions in form of changes in position of the eye.

The biosignal processing unit 150 may be configured to process the received biosignal data from each pair of electrodes 142a-b, 142c-d separately such that horizontal and vertical eye movements may be separately determined. The horizontal and vertical eye movements may then be combined into a single representation of the eye movements in two dimensions, wherein each sample provides a representation of both a horizontal and a vertical eye position in a time instance.

The biosignal processing unit 150 may also be configured to process the received biosignal data in order to detect other eye activities than eye movements. For instance, the biosignal processing unit 150 may be configured to determine closing and opening of an eyelid of the eye or saccades. The biosignal processing unit 150 may thus comprise an algorithm for analyzing the biosignal data so as to determine when an eye is about to close or when the eye is closed, when an eye is about to open or when the eye is open, or when a rapid eye movement corresponding to a saccade occurs.

The biosignal processing unit 150 may be any unit being capable of processing the biosignal data and determining eye movements and/or eye positions based on the acquired biosignal data. The biosignal processing unit 150 may be implemented as a dedicated hardware biosignal processing unit including circuitry dedicated to performing the functions of the biosignal processing unit 150. The circuit may be a digital logic circuit. The circuit may be implemented in an integrated circuit such as a chipset. The circuit may also be implemented in a FPGA or an ASIC. The biosignal processing unit 150 may also be implemented in circuitry being shared with the image processing unit 120. The biosignal processing unit 150 may also be implemented in software residing in a general-purpose processing unit, such as a microcontroller (MCU), a microprocessor, e.g. a CPU, which may be provided with a set of software instructions for performing the processing operations. The biosignal processing unit 150 may be implemented in a same processing unit as the image processing unit 120. For instance, the biosignal processing unit 150 and the image processing unit 120 may be implemented as separate processing threads, which may be executed on a common processor.

The biosignal processing unit 150 may be directly connected to the circuitry 144 of the biosignal acquisition unit 140, e.g. by being mounted on a common printed circuit board or connected through a wired connection to the circuitry 144.

Alternatively, the biosignal processing unit 150 may be arranged remotely to the biosignal acquisition unit 140. For instance, the biosignal processing unit 150 may be arranged in a desktop computer, a laptop, a TV, a video game console or in a portable computing device, which may also be carried or worn by the user, such as in a mobile phone or a tablet computer. Thus, the transmitter 130 may also be arranged for communicating between the biosignal acquisition unit 140 and the biosignal processing unit 150.

The system 100 may be arranged as a self-contained unit on a head-mounted structure. All components of the system 100 as described above may thus be arranged in a common and compact housing 102. This implies that the system 100 may be manufactured and delivered as a separate, self-contained unit, which may later be installed or integrated in or on a head-mounted structure, such as goggles, which may be separately manufactured (even at a different location from manufacturing of the system 100). For instance, the housing 102 may be attached to or mounted on frames of goggles or other eyewear to be worn by a user.

Referring now to Fig. 3, a method 200 for monitoring an eye position will be described. The method may be performed by a system 100 as described above.

The method comprises capturing 202 a sequence of digital images of an eye. The capturing 202 of the sequence of digital images may be performed by the image capturing device 110. Each digital image may thus provide a representation of a position of the eye in a time instant corresponding to the time of capturing the digital image.

The method further comprises acquiring 204 a sequence of biosignal data. The acquiring 204 of the sequence of biosignal data may be performed by the biosignal acquisition unit 140. The acquiring of the sequence of biosignal data may occur simultaneously with the capturing of the sequence of digital images of the eye. A sample in the sequence of biosignal data may provide a representation of a position of the eye or a change in the position of the eye in a time instant corresponding to the time of acquiring the sample.

The method further comprises determining 206 a set of reference eye positions based on the sequence of digital images. The sequence of digital images may be transferred to the image processing unit 120, which may be configured to extract an eye position from each image and the extracted eye positions may thus form a set of reference eye positions, each related to a time instant in which the respective digital image was captured.

The method further comprises determining 208 a set of intermediate eye positions. The determining of the set of intermediate eye positions may be based on the sequence of biosignal data, which may be processed by the biosignal processing unit 150 in order to determine relative eye positions or changes in positions of the eye. The relative eye positions may be combined with one or more reference eye positions in order to determine absolute positions of the eye based on the sequence of biosignal data in combination with the set of reference eye positions. Hence, intermediate eye positions representing eye positions between consecutive pairs of images of the sequence of digital images may be determined such that a sequence of eye positions may be generated with a higher rate than a rate of capturing digital images.

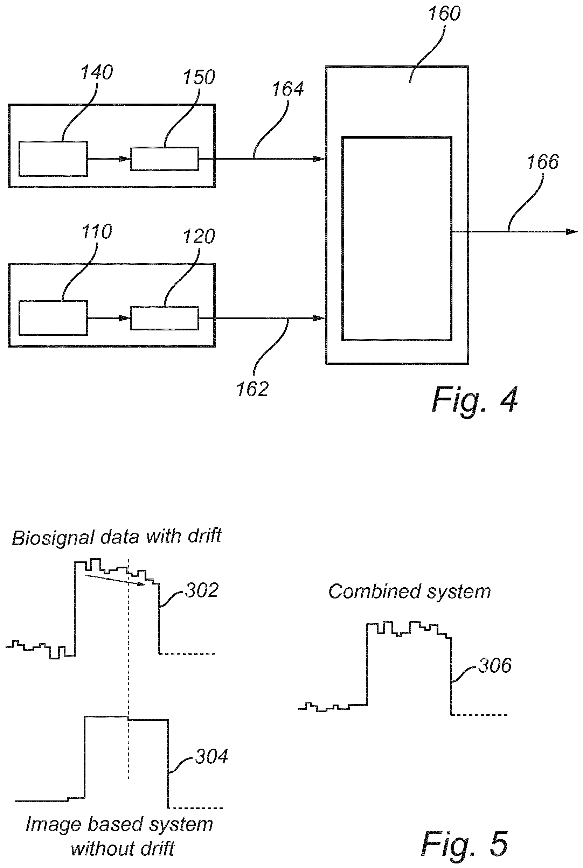

Referring now to Fig. 4, a combination of the information captured by the image capturing device 110 and the biosignal acquisition unit 140 will be further described. The image-based eye tracking and the biosignal-based eye tracking may form two parallel processes, which each acquire a set of data that may be combined to a sequence of eye positions.

The image capturing device 110 may hence transfer a sequence of digital images to the image processing unit 120. The image processing unit 120 may be configured to extract an eye position corresponding to each digital image and may hence output a set of reference eye positions, e.g. in the form of x and y coordinates in a coordinate system. The digital images may be captured with a relatively low frame rate and the reference eye positions may hence also be provided at a corresponding rate.

The image processing unit 120 may also be configured to determine other features that may be extracted from the digital image of the eye. For instance, pupil size and iris measurements may be performed for each digital image.

The image processing unit 120 may be configured to output the set of reference eye positions in a first stream 162 to a combining unit 160. Also, further features of the eye may be output with the positions of the eye in the first stream 162 of information or in a separate stream (which may facilitate extracting of the eye positions in further processing).

The biosignal acquisition unit 140 may transfer a sequence of biosignal data to the biosignal processing unit 150. The biosignal processing unit 150 may be configured to convert each sample of biosignal data to a corresponding relative eye position and may hence output a set of relative eye positions, e.g. in the form of changes in x and y coordinates in the coordinate system. The biosignal data may be captured with a relatively high frame rate and the relative eye positions may hence also be provided at a corresponding rate. The biosignal processing unit 150 may be configured to output the set of relative eye positions to the combining unit 160.

The biosignal acquisition unit 140 may be configured to acquire potential differences in two independent channels corresponding to horizontal (x) and vertical (y) movements of the eye. Thus, the relative eye positions may be based on two independent channels, which may be output in a second stream 164 comprising the determined relative eye positions.

The biosignal processing unit 150 may further be configured to perform event detection based on the biosignal data. The biosignal processing unit 150 may thus be configured to detect blinks and/or saccades and may output the events with the relative positions of the eye in the second stream 164 of information or in a separate stream (which may facilitate extracting of the eye positions in further processing). The events may be used in user interaction, as even-driven input to an eye-controlled computing device. Blinks and saccades could for instance be used as commands to the computing device, e.g. for selecting an item.

The combining unit 160 may receive the first and second streams and combine the input to a single stream 166 of eye positions. The single stream of eye positions may provide a rate which is substantially higher than the rate of the first stream 162 and may be equal to the rate of the second stream 164. The combining unit 160 may also select only parts of the relative eye positions of the second stream to be included in the outputted single stream 166. For instance, every other sample point of the second stream 164 may be included in the single stream 166, although every sample point may contribute to accuracy of the individual eye positions.

The reference eye positions and the relative eye positions may be provided to the combining unit in relation to a common coordinate system, such that the combining unit 160 may directly combine the information into a single stream. A sequence of relative eye positions corresponding to time instances between two consecutive digital images may be processed in relation to the first reference eye position for determining absolute intermediate eye positions as offsets to the first reference eye position. Thus, a set of eye positions may be formed in (near) real time.

Since the relative eye positions may be separately processed, the combining unit 160 may receive relative eye positions from the biosignal processing unit 150 as soon as they are generated. The reference eye positions may not be as quickly generated in relation to capturing of the digital image, as the image processing may require more complex processing than the biosignal data processing. As soon as the combining unit 160 receives a reference eye position, the combining unit 160 may determine intermediate eye positions based on relative eye positions, which may have already been received from the biosignal processing unit 150. The combining unit 160 may then continue to determine intermediate eye positions based on new relative eye position information received from the biosignal processing unit 150, until a new reference eye position is received from the image processing unit 120.

The reference eye positions and the relative eye positions may be associated with time stamps, so that the combining unit 160 is able to relate the first and second streams 162, 164 to each other. Alternatively, a clock is shared by the image processing unit 120 and the biosignal processing unit 150 and the output of information to the combining unit 160 may be clocked such that the combining unit 160 may use default information in order to relate the reference eye positions to the relative eye positions in time.

As mentioned above, two parallel systems 100 may also be set up to monitor the position of both eyes of a user. The systems 100 may in such case share the combining unit 160 such that the combining unit 160 receives input of first and second streams 162, 164 for each of the eyes. The combining unit 160 may thus form a single stream 166 for the right eye and another single stream for the left eye. However, it should be realized that eye positions, e.g. in the form of angles representing gaze directions for both of the eyes may be used in order to draw further conclusions on what the user looks at. For instance, by determining gaze directions of each eye, the gaze directions may be compared in order to determine a distance from a user to an object at which the user is looking. If the user is looking at an object that is close to the user, angles of the gaze directions of the respective eyes may form large converging opposite angles, whereas if the user is looking at an object far away, the gaze directions may form close to parallel angles. Such information may be determined by the combining unit 160 which may then not only output information of positions of the respective eyes but also a distance to an object that the user looks at.

It should be realized that the combination of information from parallel systems 100 may be obtained in other ways. For instance, each system 100 may comprise a combining unit 160 that outputs a single stream 166 of eye positions and another processing block, which may be arranged in an overall control system may receive the streams of eye positions for e.g. determining a distance to an object. Alternatively, it could be contemplated that the parallel systems 100 share processing units for image processing and biosignal processing for both eyes.

Thanks to use of biosignal data, there is not a need to capture images very often in order to provide a high rate of eye positions. This implies that the image capturing frame rate could be set in relation to desired accuracy and not necessarily in relation to a capability of image capturing and image processing. The capturing and processing of images may be relatively complex and, hence, power consuming, so by limiting the image capturing frame rate, power consumption may be controlled and, also, battery life of a portable device may be increased. The desired accuracy of the eye positions may be related to how fast a drift of the eye positions based on biosignal data causes errors in the eye positions to exceed a threshold.

In Fig. 5, an example illustrates that the high sampling rate of the biosignal data, shown in graph 302, offers good short term observations on the eye position with high accuracy. As time passes however, this signal starts to drift and accuracy decreases. On the other hand, the image-based determination of eye positions, shown in graph 304, does not have a drift. However, the image-based determination of eye positions has no possibility to detect short term eye movements. By combining both types of data, illustrated in graph 306, a combination of the high sampling rate and short term accuracy of the biosignal data with the long term accuracy offered by the image-based system is obtained, without the need of sampling at a high rate with the image capturing device 110.

According to an embodiment, images may be captured at a rate of two frames per second, whereas biosignal data may be acquired at a rate of 256 Hz. The single stream of eye positions may output eye positions at a rate of 256 Hz, or 128 Hz. It should be realized that any other combination of rates is conceivable and the above values should only be taken as illustrative examples.

In an embodiment, the first and second streams 162, 164 may be processed and analyzed separately from capturing of images and acquiring of biosignal data. Hence, the image processing unit 120 may determine a set of reference eye positions based on a sequence of digital images and may transfer the entire set of reference eye positions to the combining unit 160 in a single transfer of information, which may occur at any time after the capturing of the images. Similarly, the biosignal processing unit 150 may determine a set of relative eye positions based on a sequence of biosignal data and may transfer the entire set of relative eye positions to the combining unit 160 in a single transfer of information. The combining unit 160 may thus combine the reference eye positions and the relative eye positions to a single stream of eye positions. In combining the information, the relative eye positions may be related to reference eye positions both before and after in time in relation to the relative eye position. This may improve accuracy of eye positions based on the relative eye positions, especially at time instances immediately prior to a reference eye position. The eye positions thus determined are not provided in real time, but could be useful e.g. in applications of analyzing eye movements.

The combining unit 160 may be any unit being capable of processing the received first and second streams and determining eye positions based on the received data. The combining unit 160 may be implemented as a dedicated hardware combining unit including circuitry dedicated to performing the functions of the combining unit 160. The circuit may be a digital logic circuit. The circuit may be implemented in an integrated circuit such as a chipset. The circuit may also be implemented in a FPGA or an ASIC. The combining unit 160 may also be implemented in circuitry being shared with the image processing unit 120 and/or the biosignal processing unit 150. The combining unit 160 may also be implemented in software residing in a general-purpose processing unit, such as a microcontroller (MCU), a microprocessor, e.g. a CPU, which may be provided with a set of software instructions for performing the processing operations. The combining unit 160 may be implemented in a same processing unit as the image processing unit 120 and/or the biosignal processing unit 150. For instance, the combining unit 160, the image processing unit 120 and the biosignal processing unit 150 may be implemented as separate processing threads, which may be executed on a common processor.

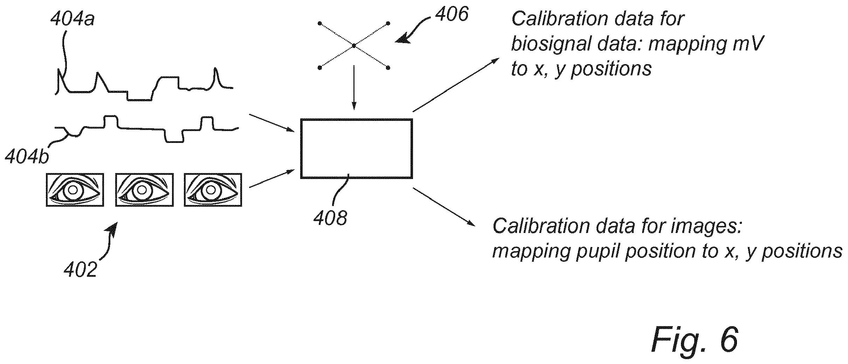

Referring now to Fig. 6, a calibration procedure will be described. Calibration may be performed in relation to a coordinate system, which is later to be used in representing the eye positions. For instance, an external coordinate system may be used, which may refer e.g. to a screen which is to be controlled by monitoring of eye movements. However, it should be realized that calibration could alternatively be performed in relation to an internal coordinate system, such as the coordinate system in an image of the eye, wherein the biosignal data may be calibrated to a sequence of known positions (e.g. by imaging) of the eye.

As illustrated in Fig. 6, the calibration procedure may comprise capturing a calibration sequence 402 of digital images of the eye and acquiring a calibration sequence 404a of biosignal data relating to eye movements in a vertical direction and a calibration sequence 404b of biosignal data relating to eye movements in a horizontal direction. The calibration sequences 402, 404a-b may be acquired in relation to predictable eye positions. For instance, stimuli trigging a sequence 406 of predictable eye positions may be provided.

The stimuli could for instance be provided as indications of screen positions, which would trigger a user to position the eye so as to be directed towards the screen position. The indications of screen positions may comprise positions at edges of the screen in order to acquire calibration sequences 402, 404a-b corresponding to large differences in eye positions.

The stimuli trigging a sequence 406 of predictable eye positions may alternatively comprise an indication, such as a dot or another pattern, on the screen, which indication is moving across the screen. The calibration procedure may include the pattern moving across the screen at several different speeds in order to acquire calibration sequences 402, 404a-b in relation to different speeds of eye movements.

The sequence 406 of predictable eye positions and the calibration sequences 402, 404a-b may be input to a calibration calculating algorithm 408. The calibration calculating algorithm may correlate positions and speeds of an eye in a digital image to corresponding screen positions and may also correlate potential differences in biosignal data to movements in screen positions.

The calibration data may thus correlate reference eye positions and relative eye positions to screen positions. The calibration data may also comprise an indication of gaze direction or angle of the eye based on a distance between the user and the screen during calibration. When the system 100 is used, a distance of the user to the screen may be determined such that an angle resolution (minimum detectable angle perceived by the system) can be computed.

The calibration data may be stored in a memory, which is accessible by the image processing unit 120 and the biosignal processing unit 150. In an embodiment, the calibration data relevant to the image-based eye tracking is stored in a memory associated with the image processing unit 120 and the calibration data relevant to the biosignal-based eye tracking is stored in a memory associated with the biosignal processing unit 150. The image processing unit 120 and the biosignal processing unit 150 may use the respective calibration data to convert the received information to eye positions in relation to screen coordinates.

The calibration procedure may be performed periodically. For instance, the calibration procedure may be performed every time the system 100 for monitoring an eye position is turned on. Also, the calibration procedure may be performed based on an internal trigger, such as detecting that a user has repositioned, that signal quality is deteriorating, etc., or based on an external trigger, such as initiated by a user.

The calibration procedure may need to be performed if a user of the system 100 is changed, as the eye may not be imaged in the same way by the image capturing device 110 (e.g. different distance between eye and the image capturing device 110) and a different response to eye movements may be acquired by the biosignal acquisition unit 140.

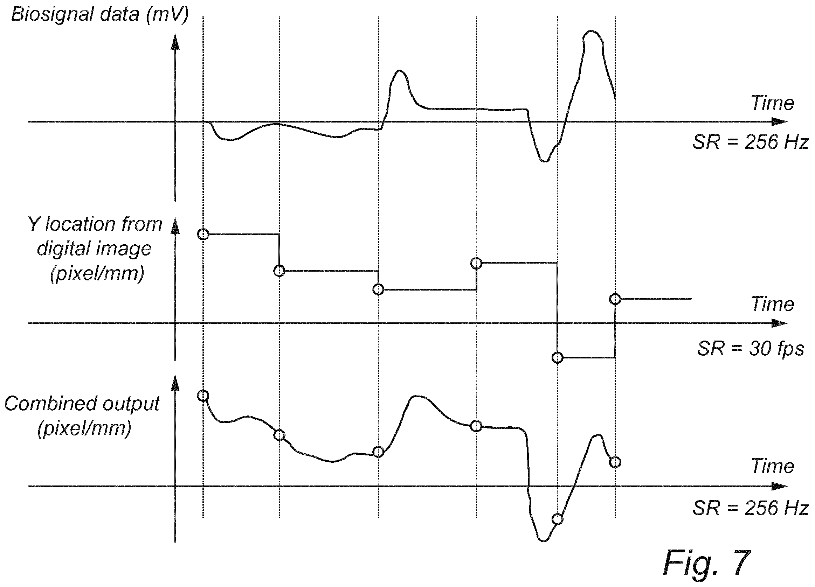

Referring now to Fig. 7, combination of image-based and biosignal-based eye tracking is illustrated. The uppermost graph illustrates biosignal data in a vertical direction (along an y axis), which provides a potential difference in dependence of eye movements. The middle graph illustrates locations along the y axis as determined based on captured images. The biosignal data is acquired with a sample rate of 256 Hz, whereas the digital images are captured with a sample rate of 30 frames per second. The eye positions based on the digital images provides reference eye positions, which are considered accurate, whereas the changes in eye positions between the reference eye positions may be determined based on the biosignal data, which is acquired at a much higher sample rate.

As shown in the lowermost graph, a combined monitoring of the eye positions provides information of intermediate eye positions between the reference eye positions, based on the biosignal data. The combined output may provide a single stream of eye positions based on a combination of the digital images and the biosignal data. A monitoring of eye positions is thus enabled at a high sample rate provided by the acquiring of biosignal data, while the eye positions based on digital images provides a periodical reference to avoid drift errors.

As is clear from Fig. 7, the biosignal data allows detecting rapid changes in eye positions, which would not be detected merely by use of the digital images of the eye. For instance, between the third and fourth eye positions determined by the image capturing device 110, an upwards eye movement followed by a downwards eye movement is performed, whereas the eye positions determined based on the digital images merely indicate a small net upward movement.

As is also clear from Fig. 7, the absolute positions determined by the digital images indicate a slow net downwards eye movement. However, the biosignal data does not indicate a net downwards eye movement and thus, if only biosignal data would be used, an absolute eye position would not be reliably determined.

Hence, as is illustrated in Fig. 7, a combination of capturing digital images of the eye and acquiring biosignal data provides both a high accuracy while enabling detection of rapid eye movements.

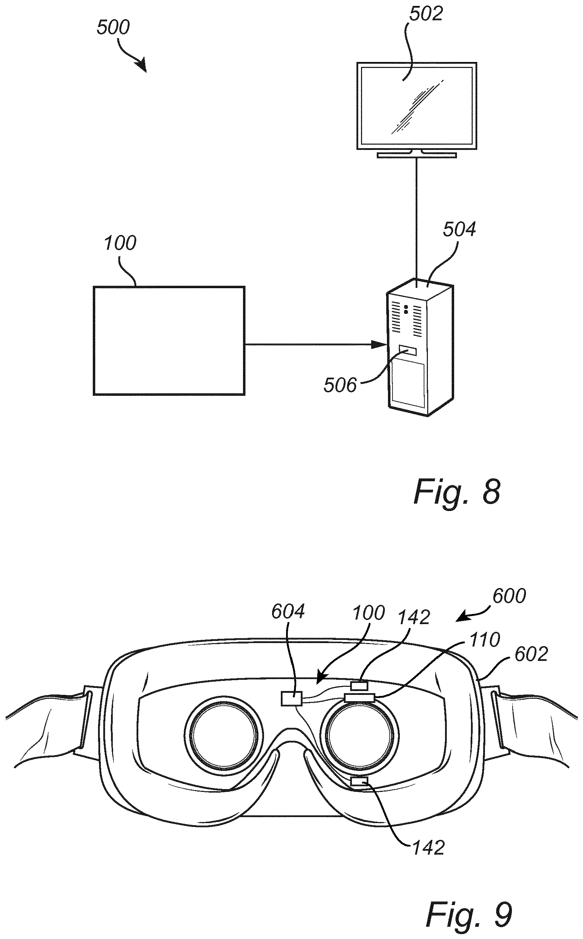

As schematically illustrated in Fig. 8, the system 100 for monitoring an eye position may be used in system 500 for controlling a presentation on a screen 502 and, hence, in interaction with a computing device 504. The screen 502 may be connected to a computing device 504, which provides output for controlling what is presented on the screen 502. The system 100 for monitoring an eye position may be calibrated to screen coordinates, such that the combining unit 160 may output a stream of eye positions providing a gaze direction of a user to indicate which coordinate (pixel) on the screen 502 the eye is directed at.

The computing device 504 may further comprise a controller 506, which may receive the stream of eye positions from the combining unit 160. Also, the controller 506 may receive information on eye events, such as blinks or saccades, which may be interpreted as commands to the computing device 504. The controller 506 may thus process the stream of eye positions in order to use the eye positions as input to the computing device 504. For instance, a cursor or pointer may follow the eye positions and a blink, when the pointer is at a desired position may be interpreted as a selection of an item presented in the position. The controller 506 may thus execute operations or cause operations of the computing device 504 to be executed in response to input based on eye positions and/or eye events. The execution of operations may then cause output of updated presentations on the screen 502 such that the presentation on the screen 502 is controlled in relation to the received eye positions.

The system 100 for monitoring an eye position may at least partly be integrated in the computing device 504. Thus, digital images captured by the image capturing device 110 and biosignal data acquired by the biosignal acquisition unit 140 may be transmitted to the computing device 504, such that the image processing unit 120, the biosignal processing unit 140 and the combining unit 160 may be integrated in the computing device 504, e.g. as a separate processing circuit or as software enabling a CPU to execute the functionalities of the image processing unit 120, the biosignal processing unit 140 and the combining unit 160.

As schematically illustrated in Fig. 9, the system 100 for monitoring an eye position may be integrated in a device 600, which may comprise a carrier 602 that is suited for mounting on a head of a user. Thus, the carrier 602 may be goggles for providing a virtual or augmented reality. The carrier 602 thus provides a well-defined relationship to the head of the user, such that when the carrier 602 is worn by the user, the carrier 602 will be arranged in relation to the eyes of the user in a predetermined way.

The image capturing device 110 may be mounted on the carrier 602 so as to be directed towards the eye of the user, which implies that high-quality images of the eye may be captured by the image capturing device 110. Further, pairs of electrodes 142 of the biosignal acquisition unit 140 may be arranged at rims of the goggles such that the electrodes 142 will be placed in contact with skin at positions close to the eye. Thus, the biosignal acquisition unit 140 will be arranged so as to acquire biosignal data representing eye movements.

Further, a processing unit 604 may be mounted at a suitable position on the carrier 602, wherein the processing unit 604 may provide the functionality of the image processing unit 120, the biosignal processing unit 150 and the combining unit 160 so as to generate a stream of eye positions. The stream of eye positions may be transmitted from the device 600 and may be used by an external computing device, e.g. to adapt a presented augmented or virtual reality based on the input provided by the eye positions.

The device 600 provides a pre-defined set-up that is easy to use and does not require cumbersome preparation of the system 100 before monitoring of eye positions may be started. The user may simply arrange the head-mounted carrier on the head, whereby the image capturing device 110 and the biosignal acquisition unit 140 will be arranged in relation to the eye in such a way as to provide data that may be used for monitoring the eye positions.

In the above the inventive concept has mainly been described with reference to a limited number of examples. However, as is readily appreciated by a person skilled in the art, other examples than the ones disclosed above are equally possible within the scope of the inventive concept, as defined by the appended claims.

For instance, eye positions are mainly presented as positions in relation to a coordinate system. The eye positions could instead be provided as angles indicating gaze directions, which may similarly provide information of the eye positions.

## Claims

1. A method for monitoring an eye position, comprising:
capturing (202) a sequence of digital images of an eye;
acquiring (204) a sequence of biosignal data representing eye movements;
determining (206) a set of reference eye positions based on the sequence of digital images; and
determining (208) a set of intermediate eye positions based on said set of reference eye positions and said sequence of biosignal data, said set of intermediate eye positions representing eye positions relative to said set of reference eye positions, wherein the set of intermediate eye positions represents eye positions between consecutive pairs of images of said sequence of digital images.

2. The method according to claim 1, further comprising determining a set of relative eye positions based on said sequence of biosignal data, wherein said determining of a set of intermediate eye positions comprises combining said set of reference eye positions and said set of relative eye positions.

3. The method according to claim 2, further comprising, in a combining unit (160), receiving a first stream (162) comprising the determined set of reference eye positions, receiving a second stream (164) comprising the determined relative eye positions, and combining the first and second streams (162, 164) into a single stream (166) of eye positions.

4. The method according to claim 3, wherein a sample rate of the second stream (164) is higher than a sample rate of the first stream (162), and wherein the combined single stream (166) has a sample rate higher than the sample rate of the first stream (162).

5. The method according to any one of claims 2-4, wherein the set of reference eye positions and the set of relative eye positions are determined in relation to a common coordinate system.

6. The method according to claim 5, wherein the common coordinate system is based on an external reference.

7. The method according to any one of the preceding claims, further comprising calibrating eye positions based on digital images of an eye to eye positions based on biosignal data, said calibrating comprising providing a sequence (406) of stimuli trigging a sequence of predictable eye positions, capturing a calibration sequence (402) of digital images of an eye in relation to the sequence of predictable eye positions; acquiring a calibration sequence (404a-b) of biosignal data representing eye movements in relation to the sequence of predictable eye positions, and determining calibration data for the reference eye positions and calibration data for the intermediate eye positions in a common coordinate system.

8. The method according to claim 7, wherein said sequence (406) of stimuli comprises indications of screen positions and wherein said calibration data correlates reference eye positions and intermediate eye positions to screen positions.

9. A system for monitoring an eye position, comprising:
an image capturing device (110) configured to capture a sequence of digital images of an eye;
a biosignal acquisition unit (140) configured to acquire a sequence of biosignal data representing eye movements;
a processing unit (120, 150, 160) configured to determine a set of reference eye positions based on the sequence of digital images; and determine a set of intermediate eye positions based on said set of reference eye positions and said sequence of biosignal data, said set of intermediate eye positions representing eye positions relative to said set of reference eye positions, wherein the set of intermediate eye positions represents eye positions between consecutive pairs of images of said sequence of digital images.

10. The system according to claim 9, wherein the processing unit (120, 150, 160) comprises an image processor (120) configured to determine a set of reference eye positions based on the sequence of digital images and a biosignal processor (150) configured to determine a set of relative eye positions based on said sequence of biosignal data.

11. The system according to claim 10, wherein the processing unit (120, 150, 160) further comprises a combiner (160) configured to determine intermediate eye positions based on said set of reference eye positions and said set of relative eye positions.

12. The system according to claim 11, wherein the combiner (160) is configured to output a single stream (166) of eye positions based on said set of reference eye positions and said set of relative eye positions.

13. A system for controlling presentation on a screen (502), comprising:
a screen (502) configured to present information to a user;
a system (100) for monitoring an eye position according to any one of claims 9-12, wherein the eye positions are determined in relation to positions on the screen (502); and
a controller (506) configured to receive the eye positions as indications of gaze directions of a user and further configured to control the screen (502) in relation to the received eye positions.

14. A device for monitoring an eye position, comprising:
a carrier (602) configured to be head-mounted on a user; and
the system (100) according to any one of claims 8-12, wherein the system (100) is mounted on the carrier (602).

15. The device according to claim 14, wherein the carrier (602) is a pair of glasses or a headset for virtual or augmented reality.
